# EUROPÄISCHE PATENTSCHRIFT

(11) **EP 0 735 882 B1**
(45) Veröffentlichungstag und Bekanntmachung des Hinweises auf die Patenterteilung: **08.05.2002**
(21) Anmeldenummer: 95905102.0
(22) Anmeldetag: 22.12.1994
(51) Int. Cl.: A61K 31/57, A61K 31/565

(54) **KOMBINATIONSPRODUKT ZUR KONTRAZEPTION ENTHALTEND PROGESTERONANTAGONISTEN UND GESTAGENEN**
COMBINATION CONTRACEPTIVE CONTAINING PROGESTERONE ANTAGONISTS AND A PROGESTOGEN
PREPARATION CONTRACEPTIVE COMBINEE CONTENANT DES ANTAGONISTES DE LA PROGESTERONE ET UN PROGESTATIF

(30) Priorität: 22.12.1993 DE 4344463
(43) Veröffentlichungstag der Anmeldung: 09.10.1996
(73) Patentinhaber: SCHERING AKTIENGESELLSCHAFT, 13353 Berlin (DE)
(72) Erfinder: STÖCKEMANN, Klaus, D-12161 Berlin (DE); CHWALISZ, Kristof, D-13503 Berlin (DE)
(86) Internationale Anmeldenummer: EP9404273
(87) Internationale Veröffentlichungsnummer: WO9517193

(56) Entgegenhaltungen:
- WO-A-93/21927
- FERTILITY AND STERILITY, 60 (4). OCT. 1993. 610-615., KEKKONEN R ET AL 'Sequential regimen of the antiprogesterone RU486 and synthetic progestin for contraception'
- FERTIL STERIL, APR 1990, 53 (4) P747-50, UNITED STATES, KEKKONEN R ET AL 'Interference with ovulation by sequential treatment with the antiprogesterone RU486 and synthetic progestin.'

## Beschreibung

Die vorliegende Erfindung betrifft ein Kombinationsprodukt aus einzelnen Dosierungseinheiten eines kompetitiven Progesteronantagonisten und dazu sequentiell vorgesehenen einzelnen Dosierungseinheiten einer Verbindung mit gestagener Wirkung sowie dessen Verwendung zur Herstellung von Kontrazeptiva auf der Basis der Hemmung der Implantation (Rezeptivitätshemmung).
Es ist bekannt, daß kompetitive Progesteronantagonisten (Antigestagene = AG's) wie z.B. RU486 (Mifepristone; 11β-[(4-N,N-Dimethylamino)-phenyl]-17β-hydroxy-17α-propinyl-4,9 (10)-estradien-3-on ) in der Lage sind, die Ovulation in verschiedenen Tierspezies und bei der Frau zu hemmen [ 1) Uilenbroek J.TH.J (1991): Hormone concentrations and ovulatory response in rats treated with antiprogestagens. Journal of Endocrinology, 129, 423-429; 2) Danforth R. et al (1989): Contraceptive Potential of RU486 by ovulation inhibition: III. Preliminary Observations on once weekly administration, Contraception, 40/2, 195-200; 3) Kekkonen R. et al. (1990): Interference with ovulation by sequential treatment with the antiprogestin RU486 and synthetic progestin. Fetility and Sterility, 53/4, 747-750; 4) Ledger WL. et al. (1992): Inhibition of ovulation by low dose mifepristone (RU486). Human Reproduction, 7/7, 945-950; 5) Nieman LK. et al. (1987): The progesterone antagonist RU486: A new potential new contraceptive agent. The New England Journal of Medicine, 316/4, 187-1991].
Ebenso kann durch AG's die Implantation eines befruchteten Eies verhindert werden (lmplantationshemmung);[ 6) Glassier A. et al. (1992): Mifepristone (RU 486) compared with high dose estrogen and progesterone for emergency postcoital contraception: New England Journal of Medicine, 8/15; 1041; 7) Puri CP. et al. (1990): Effects of a progesterone antagonist, lilopristone , on induction of menstruation, inhibition of nidation, and termination of pregnancy in bonnet monkeys. Biology of Reproduction, 43, 437-443; 8) Ishwad PC et al. (1993): Treatment with a progesterone antagonist ZK 98 299 delays endometrial development without blocking ovulation in bonnet monkeys. Contraception, 48, 57-70; 9) Batista MC. et al. (1992): Delayed endometrial maturation induced by daily administration of the antiprogestin RU 486: A potential new contraceptive strategy. AM. J. Obstet. Gynecol. 167/1, 60-65].

AG's sollten wegen ihrer ovulations- [3), 4), 5)] oder implantationshemmenden Wirkung [6), 7), 8, 9)] als Kontrazeptiva einsetzbar sein. Die Verwendung von kompetitiven Progesteronantagonisten in einer nicht-ovulationshemmenden sowie nicht-abortauslösenden Dosis zur Herstellung von oralen Kontrazeptiva ist in der internationalen Patentanmeldung WO-A 93/23020 beschrieben.

Außerdem haben für gynäkologische Anwendungen erste klinische Studien gezeigt, daß AG's für die Behandlung der Endometriose und des Leiomyomata uteri angewendet werden können [ 10) Kettel LM. et al. (1991): Endocrine responses to long-term administration of the antiprogesterone RU 486 in patients with pelvic endometriosis. Fertility and Sterility, 56/3, 402-407; 11) Kettel LM et al. (1993): Long-term, low-dose RU486 in the treatment of endometriosis. Meeting of the Society of Gynecological Investigation 1993, Abstract S-136; 12) Murphy AA et al. (1993): Regression of uterine leiomyomata in response to the antiprogestin RU486, J. Clin. Endocrinol. Metab., 76/2, 513-517].
Die Befunde aus diesen Studien deuten darauf hin, daß es bei einer chronischen Behandlung mit AG's über den gesamten Menstruationszyklus, aber auch bei der Behandlung während bestimmter Zyklusphasen mit AG's, bedingt durch die Aufhebung der Progestronwirkung während der Lutealphase des Cyclus, zu einer Verschiebung bzw. Verlängerung des Zyklus mit einem Ausbleiben der monatlichen Blutung ( Amenorrhea) bzw. abgeschwächten Menstruation kommen kann [8), 9), 10)].
Die monatliche Blutung bedeutet jedoch einen natürlichen Schutz für das Endometrium. Im normalen Menstruationszyklus kommt es in der Follikelphase (Proliferationsphase) unter der Wirkung von Östrogenen zu einer Proliferation des Endometriums. Im Anschluß daran findet in der Lutealphase (Sekretionsphase) eine durch Progesteron verursachte Wachstumsinhibition des Endometriums mit einer Umwandlung in ein sekretorisch aktives Endometrium statt. Am Ende dieser Phase kommt es zu der monatlichen Blutung des Endometriums, bei der Teile dieses Gewebes abgestoßen werden.
Wird aber während einer Behandlung mit einem kompetitiven Progesteronantagonisten die Progesteronwirkung auf das Endometrium in der Lutealphase vollständig blockiert, so dominiert der proliferative Einfluß der Östrogene auf das Endometrium. Es kann dann neben dem Ausbleiben der Umwandlung in ein sekretorisches Endometrium und der damit fehlenden [10)] anschließenden Blutung (Induktion einer Amenorrhae) bzw. einer abgeschwächten Blutung [8)] aufgrund des sogenannten "unopposed estrogen effect" zu einer Dauerstimulation des Endometriums kommen [13) Murphy AA et al. (1993): Endometrial effect of a long-term, low-dose administration of RU 486 in cycling women. Meeting of the Society of Gynecological Investigation 1993, Abstract S-138].
Dies kann das Risiko einer Endometriumshyperplasie bzw. die Entstehung eines Endometriumkarzinoms erhöhen [14) Galle PC and McRae MA (1992): Amenorrhea and chronic anovulation. Finding and adressing the underlying cause. Postrad. Med., 92/2, 255-260; 15) Johansson ED. et al. (1981): Unopposed endogenous estrogens and the incidence of cancer in female reproductive organs. Acta Obstet. Gynecol. Scand. Suppl., 101, 17-20].

Es ist bekannt, daß Gestagene, appliziert in Kombination mit einem Progesteronantagonisten, zu einer normalen Menstruation führen [3), S. 749, rechte Spalte, Discussion, 3. Absatz;17) Kekkonen R. et al. (1993): Sequential regimen of the antiprogesterone RU486 and synthetic progestin for contraception, S. 614, rechte Spalte, Z. 5, 6 und S. 615, linke Spalte, Z. 18-22] und daß Gestagene für eine Normalisierung des Endometriums verantwortlich sind [3), S. 749, rechte Spalte, Discussion, 3. Absatz]. Gemäß dieser Dokumente wird der kompetitive Progesteronantagonist in einer die Ovulation hemmenden Dosis (25 mg RU486) verabreicht. Das Gestagen muß hierbei verabreicht werden, um überhaupt eine Blutung zu induzieren.

Aufgabe der vorliegenden Erfindung ist es, die bereits bekannte Verwendung von kompetitiven Progesteronantagonisten für die Kontrazeption auf der Basis einer Rezeptivitätshemmung derart zu verbessern, daß die unerwünschten Wirkungen, die unter einer kontinuierlichen Behandlung mit kompetitiven Progesteronantagonisten, wie z.B. persistierende Amenorrhoe, Endometriumshyperplasie etc., nicht, oder nur im geringem Maße auftreten, damit eine effizientere und sicherere Behandlung sowie eine bessere Zykluskontrolle gewährleistet wird.

Diese Aufgabe wird durch das erfindungsgemäße Produkt, enthaltend in Kombination in einer Verpackungseinheit, einzelne Dosierungseinheiten eines kompetitiven Progesteronantagonisten und einzelne Dosierungseinheiten eines Gestagens zu dessen sequentieller, oraler Verabreichung, worin jede einzelne, den kompetitiven Progesteronantagonisten enthaltende Dosierungseinheit, diesen in einer nicht-ovulationshemmenden sowie nicht-abortauslösenden Menge enthält, gelöst.

Durch die sequentielle Gabe eines Gestagens während der Behandlungspause mit dem kompetitiven Progesteronantagonisten wird das Endometrium transformiert und in ein sekretorisch aktives Endometrium umgewandelt und somit auf eine - durch die anschließende Gabe des Antigestagens induzierte - Blutung vorbereitet.

Eine kontrazeptionelle Methode unter sequentieller Behandlung mit Antigestagenen zur Ovulationshemmung mit darauffolgendem Gestagen ist bereits beschrieben worden [3)]. Die die Ovulation hemmende Dosis hängt stark von der betreffenden Spezies ab und beträgt beispielsweise für RU486 bei der Frau 2 - 5 mg [ Ledger WL. et al., 4)].
Bei dem erfindungsgemäß vorgeschlagenen kontrazeptionell wirksamen Kombinationsprodukt soll der kompetitive Progesteronantagonist in einer nicht ovuiationshemmenden Dosis verabreicht werden. In diesem Fall hemmt der Progesteronantagonist u.a. die Entwicklung endometrialer Drüsen, deren Funktion für die Implantation eines befruchteten Eies im Uterus Voraussetzung ist (hemmt somit die Rezeptivität des Endometriums), interferiert aber nicht mit der Ovulation. Dadurch wird eine Beeinträchtigung des ovariellen Zyklus vermieden. Im Anschluß an die im normalen Cyclus rezeptive Phase des Endometriums, die durch kompetitiven Progesteronantagonisten gehemmt ist, wird durch das Gestagen - entsprechend dem normalen Zyklus in der Luteal-Phase - das Endometrium für eine durch den nach der Gestagengabe wieder zur Verabreichung kommenden Progesteronantagonisten induzierte Blutung vorbereitet (siehe Schema 1).
Im normalen Zyklus unterscheidet man zwei Phasen, die Proliferationsphase (Follikelphase) und die Sekretionsphase (Lutealphase). In der Follikelphase findet im normalen Menstruationszyklus eine Estrogen-induzierte Entwicklung der sekretorischen Drüsen im Endometrium statt, während in der Lutealphase das Gestagen (Progesteron) die sekretorische Aktivität der Drüsen induziert. Der Progesteronantagonist dient gemäß vorliegender Erfindung der Inhibierung sowohl der Entwicklung endometrialer Drüsen schon während der Follikelphase als auch der sekretorischen Umwandlung der Drüsen in der Lutealphase, die für eine erfolgreiche Implantation des befruchteten Eies essentiell ist. Da es unter einer Behandlung mit einem kompetitiven Progesteronantagonisten nicht zu einer vollständigen Suppression der Estrogene kommt, [die Spiegel sind vergleichbar mit denen in der mittleren Follikelphase (10)], wird im Anschluß an die Hemmung der rezeptiven Phase (nur in dieser Phase könnte - wenn sie nicht gehemmt ist - eine Implantation stattfinden), durch ein Gestagen das Endometrium (entsprechend dem normalen Zyklus) umgewandelt und auf eine der natürlichen Regel-Blutung entsprechende Blutung, die durch die fortgesetzte AG Behandlung induziert wird, vorbereitet.
Daß eine Blutung durch kompetitive Progesteronantagonisten induziert werden kann, ist beschrieben [5)]. Ebenso ist dies in Anwesentheit von Progesteron möglich [16) Croxatto HB, Spitz IM, Salvatierra AM and Bardin CW (1985). The demonstration of the antiprogestin effects of RU 486 when administered to the human during hCG-induced pseudopregnancy. In Baulieu EE. and Segal SJ (eds.). The Antiprogestin Steroid RU486 and Human Fertility Control. Plenum Press, New York, pp 263-269].
Durch die sequentielle Behandlung mit dem Gestagen wird eine adäquate Zykluskontrolle gewährleistet.

Anders als in 3) und 17) wird das sequentiell verabreichte Gestagen nicht zum Auslösen einer Blutung benötigt, sondern stellt erfindungsgemäß lediglich einen zusätzlichen Schutz für das Endometrium dar.

Mit der vorgeschlagenen Zusammensetzung lassen sich die unerwünschten Wirkungen einer möglichen Monotherapie mit einem kompetitiven Progesteronantagonisten (chronische Amenrrohea und Stimulation des Endometriums) verhindern.
Durch eine Pause in der Behandlung mit dem kompetitiven Progesteronantagonisten von 2 bis 12, vorzugsweise 5-10 Tagen, in der ein Gestagen in einer wirksamen Dosis 2 bis 12, vorzugsweise 5 bis 10, Tage lang gegeben wird, wird das Endometrium auf eine Blutung vorbereitet (Transformation in ein sekretorisches Gewebe). Die anschließende weiterführende Behandlung mit einem kompetitiven Progesteronantagonisten (nicht-ovulationshemmende Dosis) simuliert den natürlichen. Progesteronabfall (Progesteronblockade) und löst eine Menstruation aus, bei der Teile des Endometriums abgestoßen werden. Durch die regelmäßige tägliche Verabreichung eines Gestagens über einen bestimmten Zeitraum in einer wirksamen Dosis z.B. alle 28 Tage (Länge eines unbehandelten Zyklus) oder z.B. in Abständen, die 3 normalen, unbehandelten Zyklen entsprechen, wird das Endometrium auf eine Blutungs-Induktion durch einen kompetitiven Progesteronantagonisten vorbereitet. Damit wird die Manifestierung einer anhaltenden Amenorrhoea oder eine durch die fehlende Umwandlung des Endometriums in der Lutealphase verursachte Endometriumshyperplasie, die unter einer chronischen alleinigen AG-Behandlung entstehen kann, verhindert und damit eine bessere Zykluskontrolle gewährleistet. Das Endometrium wird durch eine regelmäßige Induktion der Blutung (Simulation einer natürlichen Blutung) vor oben beschriebenen Effekten geschützt.

In den Schemata 1, 2 und 3 sind exemplarisch verschiedene Ausgestaltungsmöglichkeiten der Zusammensetzung dargestellt.

Das sequentiell zum kompetitiven Progesteronantagonisten zur Verabreichung kommende Gestagen ist im erfindungsgemäßen Produkt frühestens zur Verabreichung ab dem 18. Tag nach der ersten Gabe des kompetitiven Progesteronantagonisten vorgesehen.
Die Anzahl der zu verabreichenden Dosierungseinheiten des kompetitiven Progesteronantagonisten sowie die Anzahl der anschließend täglich zu verabreichenden gestagenhaltigen Dosierungseinheiten kann so gewählt werden, daß die durch die Gestagengabe ausgelöste Menstruationsblutung zeitlich der Menstruationsblutung in einem unbehandelten Zyklus entspricht (Schema1)).
Die erfindungsgemäße Zusammensetzung kann die sequentiell zum kompetitiven Progesteronantagonisten zu verabreichende Dosierungseinheiten des Gestagens auch so angeordnet enthalten, daß sie spätestens nach einer 3 mal 28tägigen (entspricht der Dauer von 3 normalen, unbehandelten Zyklen) Gabe des Progesteronantagonisten vorgesehen sind (Schema 3)).
Zwischen diesen beiden Grenzen (kompetitiver Progesteronantagonist über einen Zeitraum von18 bzw. 3 mal 28 Tagen) sind alle denkbaren Fälle möglich, also z.B. 2 mal 28 Tage Gabe des kompetitiven Progesteronantagonisten, danach Gestagengabe.
Als kompetitive Progesteronantagonisten gemäß vorliegender Erfindung kommen alle Verbindungen in Betracht, die selbst oder deren metabolische Produkte die Wirkung des Progesterons an dessen Rezeptor blockieren. Als typische Vertreter seien hier stellvertretend genannt:
11 β-((4-N,N-Dimethylamino)-phenyl)-17β-hydroxy-17α-propinyl-4,9 (10)-estradien-3-on (RU-38486),
11β-((4-N,N-Dimethylamino)-phenyl)-17β-hydroxy-18-methyl-17α-propinyl-4,9 (10)-estradien-3-on,
11β-((4-N,N-Dimethylamino)-phenyl)-17αβ-hydroxy-17aα-propinyl-D-homo-4,9 (10),16-estratien-3-on,
11β-p-Methoxyphenyl-17β-hydroxy-17α-ethinyl-4,9 (10)-estradien-3-on
11β-(4-Acetylphenyl)-17β-hydroxy-17α-(prop-1-inyl)-4,9 (10)-estradien-3-on
11β-[(4-Dimethylamino)phenyl]-17α-hydroxy-17β-(3-hydroxypropyl)-13α-methyl-4,9(10)-gonadien-3-on (Onapriston
11β,19-(4-Acetylphenyl)-17β-hydroxy-17α-(3-hydroxyprop-1(Z)-enyl)-4,9(10)-estradien-3-on
11β,19-(4-(Cyanphenyl)-17β-hydroxy-17α-(3-hydroxyprop-1(Z)-enyl)-4-androsten-3-on
11β,19-(4-(3-Pyridinyl)-o-phenylen)-17β-hydroxy-17a-(3-hydroxyprop-1 (Z)-enyl)-4-androsten-3-on.

Diese Aufzählung ist nicht abschließend; auch andere in den genannten Veröffentlichungen beschriebene kompetitive Progesteronantagonisten sowie solche aus hier nicht genannten Veröffentlichungen sind geeignet.
Für die vorliegende Erfindung sind insbesondere solche kompetitiven Progesteronantagonisten geeignet, die peripher selektiv wirksam sind, d.h. bei denen die endometriale Wirkung ausgeprägt ist, während bei der betreffenden Dosis keine oder nur geringe zentrale Wirkung über die hypophysär-ovarielle Achse beobachtet wird (siehe WO-A 93/23020).
Für die Zwecke der vorliegenden Erfindung können die kompetitiven Progesteronantagonisten lokal, topisch, enteral, transdermal oder oder parenteral appliziert werden.
Für die bevorzugte orale Applikation kommen insbesondere Tabletten, Dragées, Kapseln, Pillen, Suspensionen oder Lösungen in Frage, die in üblicher Weise mit den in der Galenik gebräuchlichen Zusätzen und Trägersubstanzen hergestellt werden können.
Die Formulierung des kompetitiven Progesteronantagonisten erfolgt analog wie sie beispielsweise für RU 486 bekannt ist.
Für die lokale oder topische Anwendung kommen beispielsweise Vaginalzäpfchen, Vaginalgels, Implantate, Vaginalringe oder transdermale Systeme wie Hautpflaster in Frage.
Eine Dosierungseinheit enthält etwa 0,1 bis 50 mg 11β-[(4-Dimethylamino)phenyl]-17α-hydroxy-17β-(3-hydroxypropyl)-13α-methyl-4,9(10)-gonadien-3-on (Onapriston = peripher selektiver, dissoziierter kompetitiver Progesteronantagonist), 0,1 - 5,0 mg 11β-((4-N,N-Dimethylamino)-phenyl)-17β-hydroxy-17α-propinyl-4,9 (10)-estradien-3-on (RU 486 = nicht-dissoziierter kompetitiver Progesteronantagonist) oder eine biologisch äquivalente Menge eines anderen kompetitiven Progesteronantagonisten.

Erfolgt die Applikation des erfindungsgemäß zu verwendenden kompetitiven Progesteronantagonisten durch ein Implantat, einen Vaginalring oder ein transdermales System, so müssen diese Applikationssysteme derart ausgebildet sein, daß die durch sie täglich freigesetzte Dosis des kompetitiven Progesteronantagonisten in diesem Bereich von 0,25 bis 50 mg 11β-[(4-Dimethylamino)phenyl]-17α-hydroxy-17β-(3-hydroxypropyl)-13α-methyl-4,9(10)-gonadien-3-on oder einer wirkequivalenten Dosis eines anderen Progesteronantagonisten liegt.
Die erfindungsgemäß zu applizierende Dosis eines kompetitiven Progesteronantagonisten liegt im nicht-ovulationshemmenden sowie nicht-abortauslösenden Dosisbereich des betreffenden Progesteronantagonisten.
Bei der Verwendung peripher selektiver kompetitiver Progesteronantagonisten können 0,1 - 50 mg pro einmaliger Gabe dosiert werden, da die peripher selektiven Substanzen eine weit höhere Dosierung zulassen, ohne daß es zur Ovulationshemmung kommt.
Einmalige Gabe soll auch bedeuten, daß bei Verwendung eines den kompetitiven Progesteronantagonisten kontinuierlich freisetzenden Applikationssystems 0,25 - 20 mg bzw. 0,1 bis 50 mg jeweils pro Tag freigesetzt werden.
Vorzugsweise ist das erfindungsgemäße Produkt so aufgebaut, daß die Applikation der einzelnen Dosierungseinheiten des kompetitiven Progesteronantagonisten alle 4 bis alle 10 Tage erfolgen kann und zwar beginnend an einem beliebigen Tag vor dem Ovulationszeitpunkt im ersten Applikationszyklus. Dadurch ist die kontrazeptive Sicherheit bereits im ersten Applikationszyklus gewährleistet (Bygdeman).
Die zeitlichen Abstände zwischen den Gaben der einzelnen Dosierungseinheiten sollen dabei vorzugsweise konstant sein.
Insbesondere sieht das erfindungsgemäße Produkt eine Applikation der jeweiligen Dosierungseinheiten des kompetitiven Progesteronantagonisten so vor, daß diese einmal pro Woche jeweils am selben Wochentag appliziert werden, beispielsweise an einem Montag ("Montagspille").
Durch den wöchentlichen Applikationsrythmus zum jeweils selben Wochentag ist eine hohe Einnahmesicherheit gewährleistet.
Das erfindungsgemäße Produkt kann auch die Applikation der einzelnen Dosierungseinheiten des kompetitiven Progesteronantagonisten täglich, jeden zweiten oder jeden dritten Tag vorsehen.

Wirkequivalenter Dosismengen verschiedener kompetitiver Progesteronantagonisten werden im Test auf antigestagene Wirkung am Kaninchen (Aufhebung der Endometriumstransformation) bestimmt.

Als Gestagene kommen gemäß vorliegender Erfindung alle Verbindungen in Frage, die aufgrund ihrer gestagenen Wirksamkeit zur Verwendung in oralen Kontrazeptiva geeignet sind. Eine Aufstellung derartiger Verbindungen findet sich bei B. Runnebaum et al., "Female Contraception: Up-date and Trends", Springer-Verlag, Berlin, 1988, Seiten 64 - 90, 109 - 121, 122 - 128 und 129 - 140.
Bevorzugte Gestagene im Rahmen vorliegender Erfindung sind Gestoden, Progesteron, Levonorgestrel, Cyproteronacetat, Chlormadinonacetat, Drospirenon (Dihydrospirorenon), Norethisteron, Norethisteronacetat, Norgestimat, Desogestrel oder 3-Ketodesogestrel.
Das Gestagen liegt im Produkt gemäß vorliegender Erfindung in einer zur oralen Applikation geeigneten Dosierungsform vor, nämlich als Tablette, Dragée, Kapsel oder Pille.
Die Formulierung des Gestagens erfolgt dabei in einer zur Herrichtung von Gestagenen für die hormonale Kontrazeption analogen Weise unter Verwendung der hierfür üblichen Hilfsstoffe.
Eine tägliche Dosierungseinheit des Gestagens enthält dieses in einer Dosis von 0,6 - 6,0 mg Levonorgestrel, 2 - 20 mg Cyproteronacetat, 0,3 - 3,0 mg Gestoden oder 0,2 - 2,0 mg Desogestrel oder eine zu diesen Dosierungen wirkequivalente Menge eines anderen Gestagens.
Zur Bestimmung wirkequivalenter Dosismengen verschiedener Gestagene wird nach bekannten Methoden vorgenommen; nähere Einzelheiten finden sich beispielsweise in den beiden Artikeln "Probleme der Dosisfindung: Sexualhormone"; F. Neumann et al. in "Arzneimittelforschung" (Drug Research) 27, 2a, 296 - 318 (1977) sowie "Aktuelle Entwicklungen in der hormonalen Kontrazeption"; H. Kuhl in "Gynäkologe" 25: 231 - 240 (1992).

### Beispiele:

***Einmal pro 28-tägigem Zyklus mehrere Tage Gestagen:***
1) 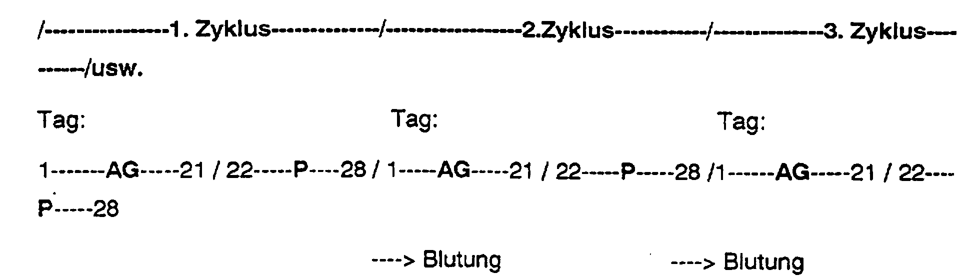
2)

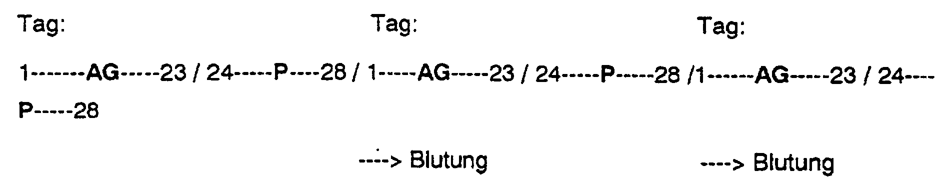

***Einmal nach drei Zyklen (1 Zyklus = 28 Tage) Gestagen für mehrere Tage:***
3)

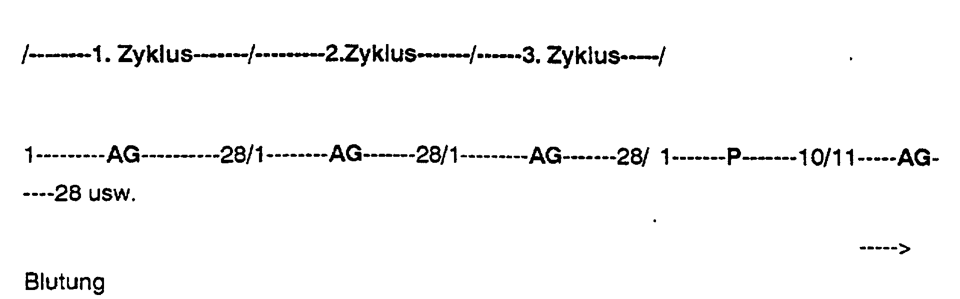

**AG steht für "kompetitiver Progesteronantagonist" und P für "Gestagen"**

## Patentansprüche

1. Produkt, enthaltend in Kombination einzelne Dosierungseinheiten eines kompetitiven Progesteronantagonisten und einzelne Dosierungseinheiten eines Gestagens zu dessen sequentieller, oraler Verabreichung, worin jede einzelne, den kompetitiven Progesteronantagonisten enthaltende Dosierungseinheit, diesen in einer nicht-ovulationshemmenden soune nicht-abortauslösenden Menge enthält.

2. Produkt nach Anspruch 1, worin jede einzelne, den kompetitiven Progesteronantagonisten enthaltende Dosierungseinheit zur einmal täglichen bis zur einmal wöchentlichen Applikation vorgesehen ist.

3. Produkt nach Anspruch 2, worin die Dosierungseinheiten des kompetitiven Progesteronantagonisten zur einmal täglichen Applikation vorgesehen sind.

4. Produkt nach Anspruch 2, worin die Dosierungseinheiten des kompetitiven Progesteronantagonisten zur einmal wöchentlichen Applikation vorgesehen sind.

5. Produkt nach Anspruch 1, worin die Dosierungseinheiten des Gestagens zur täglichen Applikation an aufeinanderfolgenden Tagen vorgesehen sind.

6. Produkt nach Anspruch 5, worin 2 bis 12 Dosierungseinheiten des Gestagens enthalten sind.

7. Produkt nach Anspruch 6, worin die Dosierungseinheiten des Gestagens sequentiell ab Tag 18 oder einem späteren Tag nach der ersten vorgesehenen Dosierungseinheit des kompetitiven Progesteronantagonist enthalten sind.

8. Produkt nach Anspruch 7, worin die Dosierungseinheiten des Gestagens ab Tag 21 oder 22 enthalten sind.

9. Produkt nach einem der vorhergehenden Ansprüche, worin die den kompetitiven Progesteronantagonisten enthaltenden Dosierungseinheiten mindestents eine Verbindung aus der
11β-((4-N,N-Dimethylamino)-phenyl)-17β-hydroxy-17α-propinyl-4,9(10)-estradien-3-on (RU-38486),
11β-((4-N,N-Dimethylamino)-phenyl)-17β-hydroxy-18-methyl-17α-propinyl-4,9 (10)-estradien-3-on,
11β-((4-N,N-Dimethylamino)-phenyl)-17αβ-hydroxy-17aα-propinyl-D-homo-4,9 (10),16-estratien-3-on,
11β-p-Methoxyphenyl-17β-hydroxy-17α-ethinyl-4,9 (10)-estradien-3-on,
11β-(4-Acetylphenyl)-17β-hydroxy-17α-(prop-1-inyl)-4,9(10)-estradien-3-on,
11β-[(4-Dimethylamino)phenyl]-17α-hydroxy-17β-(3-hydroxypropyl)-13α-methyl-4,9(10)-gonadien-3-on (Onapriston);
11β,19-(4-Acetylphenyl)-17β-hydroxy-17α-(3-hydroxyprop-1 (Z)-enyl)-4,9(10)-estradien-3-on,
11β,19-(4-(Cyanphenyl)-17β-hydroxy-17α-(3-hydroxyprop-1(Z)-enyl)-4-androsten-3-on,
11β,19-(4-(3-Pyridinyl)-o-phenylen)-17β-hydroxy-17a-(3-hydroxyprop-1(Z)-enyl)-4-androsten-3-on,
umfassenden Gruppe enthalten.

10. Produkt nach einem der vorhergehenden Ansprüche, worin in jeder einzelnen, den kompetitiven Progesteronantagonisten enthaltenden Dosierungseinheit, dieser in einer Menge von 0,1 bis 50 mg 11β-[(4-Dimethylamino)phenyl]-17α-hydroxy-17β-(3-hydroxypropyl)-13α-methyl-4,9(10)-gonadien-3-on (Onapriston = peripher selektiver, dissoziierter kompetitiver Progesteronantagonist), 0,1 - 5,0 mg 11β-((4-N,N-Dimethylamino)-phenyl)-17β-hydroxy-17α-propinyl-4,9 (10)-estradien-3-on (RU 486 = nicht-dissoziierter kompetitiver Progesteronantagonist) oder einer biologisch äquivalenten Menge eines anderen kompetitiven Progesteronantagonisten enthalten ist.

11. Produkt nach einem der vorhergehenden Ansprüche, worin die das Gestagen enthaltenden Dosierungseinheiten mindestens eine Verbindung aus der Gestoden, Progesteron, Levonorgestrel, Cyproteronacetat, Chlormadinonacetat, Drospirenon (Dihydrospirorenon), Norethisteron, Norethisteronacetat, Norgestimat, Desogestrel, 3-Ketodesogestrel oder ein anderes künstliches oder natürliches Gestagen umfassenden Gruppe enthalten.

12. Produkt nach einem der vorstehenden Ansprüche, worin in jeder einzelnen, das Gestagen enthaltenden Dosierungseinheit, dieses in einer Menge von 0,6 - 6,0 mg Levonorgestrel, 2 - 20 mg Cyproteronacetat, 0,3 - 3,0 mg Gestoden oder 0,2 - 2,0 mg Desogestrel oder einer wirkequivalenten Menge eines anderen Gestagens enthalten ist.

## Claims

1. A product that contains in combination individual dosage units of a competitive progesterone antagonist and individual dosage units of a gestagen for its sequential, oral administration, in which case each individual dosage unit that contains the competitive progesterone antagonist contains the latter in a non-ovulation-inhibiting as well as a non-abortion-inducing amount.

2. The product according to claim 1, wherein each individual dosage unit that contains the competitive progesterone antagonist is intended for once-daily to once-weekly administration.

3. The product according to claim 2, wherein the dosage units of the competitive progesterone antagonist are intended for once-daily administration.

4. The product according to claim 2, wherein the dosage units of the competitive progesterone antagonist are intended for once-weekly administration.

5. The product according to claim 1, wherein the dosage units of the gestagen are intended for daily administration on successive days.

6. The product according to claim 5, containing 2 to 12 dosage units of gestagen.

7. The product according to claim 6, containing the dosage units of the gestagen sequentially starting from day 18 or a later day after the first presecribed dosage unit of the competitive progesterone antagonist.

8. The product according to claim 7, containing the dosage units of the gestagen starting from day 21 or 22.

9. The product according to one of the preceding claims, wherein the dosage units that contain the competitive progesterone antagonist contain at least one compound selected from the group consisting of
11β-((4-N,N-dimethylamino)phenyl)-17β-hydroxy-17α-propynyl-4,9(10)-estradien-3-one (RU-38486),
11β-((4-N,N-dimethylamino)phenyl)-17β-hydroxy-18-methyl-17α-propynyl-4,9(10)-estradien-3-one,
11β-((4-N,N-dimethylamino)phenyl)-17αβ-hydroxy-17aα-propynyl-D-homo-4,9(10),16-estratien-3-one,
11β-p-methoxyphenyl-17β-hydroxy-17α-ethynyl-4,9(10)-estradien-3-one,
11β-(4-acetylphenyl)-17β-hydroxy-17α-(prop-1-ynyl)-4,9(10)-estradien-3-one,
11β-[(4-dimethylamino)phenyl]-17α-hydroxy-17β-(3-hydroxypropyl)-13α-methyl-4,9(10)-gonadien-3-one (onapristone),
11β-19-(4-acetylphenyl)-17β-hydroxy-17α-(3-hydroxyprop-1(Z)-enyl)-4,9(10)-estradien-3-one,
11β,19-(4-(cyanophenyl)-17β-hydroxy-17α-(3-hydroxyprop-1(Z)-enyl)-4-androsten-3-one,
11β,19-(4-(3-pyridinyl)-o-phenylene)-17β-hydroxy-17a-(3-hydroxyprop-1(Z)-enyl)-4-androsten-3-one.

10. The product according to one of the preceding claims, wherein each individual dosage unit that contains the competitive progesterone antagonist contains the latter in an amount of 0.1 to 50 mg of 11β-[(4-dimethylamino)phenyl]-17α-hydroxy-17β-(3-hydroxypropyl)-13α-methyl-4,9(10)-gonadien-3-one (onapristone = peripherally selective, dissociated competitive progesterone antagonist), 0.1-50 mg of 11β-((4-N,N-dimethylamino)phenyl)-17β-hydroxy-17α-propynyl-4,9(10)-estradien-3-one (RU 486 = non-dissociated competitive progesterone antagonist) or a biologically equivalent amount of another competitive progesterone antagonist.

11. The product according to one of the preceding claims, wherein the dosage units that contain the gestagen contain at least one compound selected from the group consisting of gestodene, progesterone, levonorgestrel, cyproterone acetate, chlormadinone acetate, drospirenone (dihydrospirorenone), norethisterone, norethisterone acetate, norgestimate, desogestrel, 3-ketodesogestrel or another artificial or natural gestagen.

12. The product according to one of the preceding claims, wherein each individual dosage unit that contains the gestagen contains the latter in an amount of 0.6-6.0 mg of levonorgestrel, 2-20 mg of cyproterone acetate, 0.3-3.0 mg of gestodene, or 0.2-2.0 mg of desogestrel or a biologically equivalent amount of another gestagen.

## Revendications

1. Produit contenant une combinaison de différentes unités de dosage d'un antagoniste compétitif de progestérone et de différentes unités de dosage d'un progestatif pour son administration séquentielle par voie orale, dans lequel chaque unité différente de dosage qui contient l'antagoniste compétitif de progestérone contient celui-ci en n'empêchant pas l'ovulation ainsi qu'une quantité non abortive.

2. Produit selon la revendication 1, dans lequel chaque unité différente de dosage contenant l'antagoniste compétitif de progestérone a été prévue pour une administration à raison d'une fois par jour à une fois par semaine.

3. Produit selon la revendication 2, les unités de dosages de l'antagoniste de progestérone étant prévues pour une administration à raison d'une fois par jour.

4. Produit selon la revendication 2, dans lequel les unités de dosages de l'antagoniste de progestérone ont été prévues pour une administration à raison d'une fois par semaine.

5. Produit selon la revendication 1, dans lequel les unités de dosages du progestatif ont été prévues pour une administration à raison d'une fois par jour sur des jours consécutifs.

6. Produit selon la revendication 5, dans lequel 2 à 12 unités de dosage du progestatif sont contenues.

7. Produit selon la revendication 6, dans lequel les unités de dosage du progestatif sont contenues séquentiellement à partir du 18ème jour ou d'un jour plus tard après la première unité de dosage prévue de l'antagoniste compétitif de progestérone.

8. Produit selon la revendication 7, dans lequel les unités de dosage du progestatif sont contenues à partir du 21ème ou du 22ème jour.

9. Produit selon l'une quelconque des revendications précédentes, dans lequel les unités de dosage qui contiennent l'antagoniste compétitif de progestérone contiennent au moins un composé du groupe
11β-((4-N,N-diméthylamino)phényl)-17β-hydroxy-17α-propynyl-4,9(10)estradièn-3-one (RU-38486),
11β-((4-N,N-diméthylamino)phényl)-17β-hydroxy-18-méthyl-17α-propynyl-4,9(10)estradièn-3-one,
11β-((4-N,N-diméthylamino)phényl)-17αβ-hydroxy-17aα-propynyl-D-homo-4,9(10),16-estratièn-3-one,
11β-p-méthoxyphényl-17β-hydroxy-17α-éthynyl-4,9(10)-estradièn-3-one,
11β-(4-acétylphényl)-17β-hydroxy-17α-(prop-1-ynyl)-4,9(10)-estradièn-3-one,
11β-[(4-diméthylamino)phényl]-17α-hydroxy-17β-(3-hydroxypropyl)-13α-méthyl-4,9(10)-gonadièn-3-one (Onapriston),
11β,19-(4-acétylphényl)-17β-hydroxy-17α-(3-hydroxyprop-1(Z)-ényl)-4,9(10)-estradièn-3-one,
11β,19-(4-cyanophényl)-17β-hydroxy-17α-(3-hydroxyprop-1(Z)-ényl)-4-androstèn-3-one,
11β,19-(4-(3-pyridinyl)-o-phénylène)-17β-hydroxy-17a-(3-hydroxyprop-1(Z)-ényl)-4-androstèn-3-one.

10. Produit selon l'une quelconque des revendications précédentes, dans lequel chaque unité différente de dosage qui contient l'antagoniste compétitif de progestérone contient celui-ci en une quantité de 0,1 à 50 mg de 11β-[(4-diméthylamino)phényl]-17α-hydroxy-17β-(3-hydroxypropyl)-13α-méthyl-4,9(10)-gonadièn-3-one (Onapriston antagoniste compétitif de progestérone dissocié périphériquement sélectif), 0,1 à 50 mg de 11β-((4-N,N-diméthylamino)-phényl)-17β-hydroxy-17α-propynyl-4,9(10)estradièn-3-one (RU-486 antagoniste compétitif de progestérone non dissocié) ou en une quantité biologiquement équivalente d'un autre antagoniste compétitif de progestérone.

11. Produit selon l'une quelconque des revendications précédentes, dans lequel les unités de dosage qui contiennent le progestatif contiennent au moins un composé parmi le gestodène, la progestérone, le lévonorgestrel, l'acétate de cyprotérone, l'acétate de chlormadinone, la drospirénone (dihydrospirorénone), la noréthistérone, l'acétate de noréthistérone, le norgestimate, le désogestrel, le 3-cétodésogestrel ou un autre progestatif synthétique ou naturel.

12. Produit selon l'une quelconque des revendications précédentes, dans lequel chaque unité différente de dosage qui contient le progestatif contient celui-ci en une quantité de 0,6 à 6,0 mg de lévonorgestrel, en une quantité de 2 à 20 mg d'acétate de cyprotérone, en une quantité de 0,3 à 3,0 mg de gestodène ou en une quantité de 0,2 à 2,0 mg de désogestrel ou en une quantité active équivalente d'un autre progestatif.
